# EUROPEAN PATENT APPLICATION

(11) **EP 4 364 708 A1**
(43) Date of publication of application: **08.05.2024**
(21) Application number: 22205940.4
(22) Date of filing: 07.11.2022
(51) Int. Cl.: A61F 13/49, A61F 13/551, A61F 13/56

(54) **ARRAY OF ABSORBENT ARTICLES HAVING WAIST GASKETING ELEMENTS**

(71) Applicant: The Procter & Gamble Company, Cincinnati, OH 45202 (US)
(72) Inventor: Rinnert, Thorsten, 65824 Schwalbach am Taunus (DE)
(74) Representative: P&G Patent Germany

(57) **Abstract**

An array of personal hygiene articles (10) comprising a least a first article and a second article, the second article having a larger size than the first article. The articles each comprise a back waist gasketing element (70) between a pair of elastic back ears (32). The back ears (42) each have an innermost edge (420) having a length (60). The waist gasketing element (70) has a length (70) measured along the longitudinal centerline. The ratio of the length (60) of the innermost edge of the back ears to the length of the waist gasketing element is in the range of from 1.0 to 3.0 for at least the first article and the second article. The length of the waist gasketing element of the second article is at least 2 mm longer than the length of the waist gasketing element of the first article. At least a third article having a larger size than the second article may be comprised in the array.

## Description

### FIELD OF THE INVENTION

The present disclosure relates to disposable absorbent articles, such as diapers, having a waist gasketing element capable of containing body exudates, such as fecal matter. The absorbent articles may be comprised in an array of articles of different sizes.

### BACKGROUND OF THE INVENTION

Diapers and other similar personal hygiene absorbent articles are typically built from a chassis comprising a topsheet, an acquisition layer, an absorbent core and a backsheet. Other components that include elastic parts are attached to the chassis. Most diapers have inner and outer elastic leg cuffs disposed along their longitudinal edges to improve fit around the legs of the wearer. Taped diapers further have a pair of back ears each fitted with a fastening tab that can fasten to the front of the article, thus forming a waist opening around the waist of the wearer. These back ears are typically elastic components, such as a laminate comprising an elastic film, and are attached to the chassis of the article at their innermost edge by adhesive and/or mechanical bonding.

Some taped diapers have elastic waistbands disposed along their back waist edges. These elastic waistbands are typically made from a laminate comprising an elastic film or elastic strands, which are sandwiched between two nonwoven layers. Such elastic waistbands are disposed within the chassis of the article or attached on the internal of external side of the article. Such elasticized waistbands provide increased pressure between the skin and the article. This improves containment of exudates in the back waist area. Elasticized waistbands are particularly useful to close the waist gaps that may otherwise be formed when a wearer changes positions, such as bending forward or resting on its back as for a sleeping baby.

More recently, waist gasketing elements disposed adjacent the back edges of diapers have been proposed. These waist gasketing elements can be made of similar material than elasticized waistbands, and are also placed adjacent the back waist edge of the diaper. The waist gasketing elements, also called waist cuffs, differ however from elasticized waistbands in that they are disposed on the internal side of the absorbent article and in that the innermost edge of the waist gasketing element is not attached to the chassis of the diaper, but instead can be decoupled to a certain extent from the topsheet. The other edges of the waist gasketing element are at least partially bonded to the chassis of the diaper. In this way, the waist gasketing element can form a pocket that can contain fecal matter, and/or reduce leakage of urine through the back waist edge of the article.

Waist gasketing elements can be attached to the inner surface of the article (typically the topsheet) using a variety of means, including mechanically attachment, adhesive attachment, thermal attachment, co-forming, and so forth. Recent publications WO2020/242714, WO2021/092606, WO2020/242715 and WO2021/092607 assigned to P&G disclose a waist gasketing element having at least a portion of the waist gasketing element adhesively attached to the chassis and at least a portion of the waist gasketing element mechanically attached to the chassis. The waist gasketing element thus comprises an adhesive zone, a first mechanical attachment in a non-adhesively bonded zone, a second mechanical attachment in a second non-adhesively zone, and a free standing edge.

Many absorbent articles, especially diapers, are developed as an array comprising differently sized articles, with each size adapted for a different stage of physical development of the prospective user. This is typically the case for baby diapers, where each size is typically indicated by a number, for example 0 or 1 for a new-born and up to size 6 or 7 for older infants. The size may additionally or alternatively be indicated by other signs such as letters (e.g. XS, S, M, L, XL ...) or descriptive terms ("new born", "small", "medium", "large", "extra large"..). Each size is further correlated to a pre-determined weight range. The manufacturers adapt the articles within an array of such articles by adapting the absorbent article's dimensions and absorbent capacity to the prospective users. While the numbering and corresponding weight ranges may of course differ between manufacturers, such arrays of articles of progressing sizes allow caretakers to easily switch from one article to the next one as the baby develops.

There has been until low little attention on how the dimensions of the back waist gasketing elements should be adapted according to the size of the article to provide maximum benefits. Rather, cost reasoning and rationalization have incentivized manufacturers to use waist gasketing elements of standard dimensions across the articles in a given array.

### SUMMARY OF THE INVENTION

In one aspect, the present invention is directed to an array of personal hygiene articles. The articles each comprise a chassis, a pair of back ears extending outwardly from the chassis and a waist gasketing element disposed between the back ears on the wearer-facing side of the chassis. The array comprises at least a first article having a first size, and a second article having a second size, the second article having a larger size than the first article. The size of the article may be indicated by an indicia such as a numeral and/or by a weight range for a pre-determined targeted user. This indicia is typically found on the package and/or the article itself, for example as a print on the backsheet. Each of the back ears has an innermost edge which is closest to the longitudinal centerline, this innermost edge having a length as measured in the longitudinal direction. The waist gasketing element has length measured along the longitudinal centerline of the article in the longitudinal direction.

In an aspect of the invention, it was found that the ratio of the length of the innermost edge of the back ears to the length of the waist gasketing element should be in the range of from about 1.0 to about 3.0 at least for the first article and the second article, and optionally for at least a third article in the array, and optionally for all the articles comprised in the array. In a further aspect of the invention, the length of the waist gasketing element of the second article is at least 2 mm longer than the length of the waist gasketing element of the first article. The length of the waist gasketing element of the second article may be at least at least 4 mm longer than the length of the waist gasketing element of the first article.

The waist gasketing element may comprise a proximal end edge attached to the chassis, a distal end edge that can partially stand up and away from the chassis, a first side edge, a second side edge, and an elastic material. At least a portion of the waist gasketing element may be adhesively attached to the chassis and at least a portion of the waist gasketing element may be mechanically attached to the chassis. The waist gasketing element may comprise an adhesive zone, a first adhesive-free zone and a second adhesive-free zone.

The disposable absorbent article may typically also comprise a leg gasketing system comprising longitudinally-extending inner leg cuffs and longitudinally-extending outer leg cuffs.

### BRIEF DESCRIPTION OF THE DRAWINGS

Figure 1 is a simplified front perspective view of a partially fastened taped diaper having a back waist gasketing element;
Figure 2 is a plan view of the wearer-facing surface of the absorbent article of Fig. 1, in a flat laid-out state;
Figure 3 is a plan view from the wearer-facing (internal) side of the of the back waist region of the diaper, with the waist gasketing element stretched flat;
Figure 4 is a plan view from the garment-facing (external) side of the of the back waist region of the diaper;
Figure 5 illustrates a package of diapers.

### DETAILED DESCRIPTION OF THE INVENTION

### General Description of an Absorbent Article

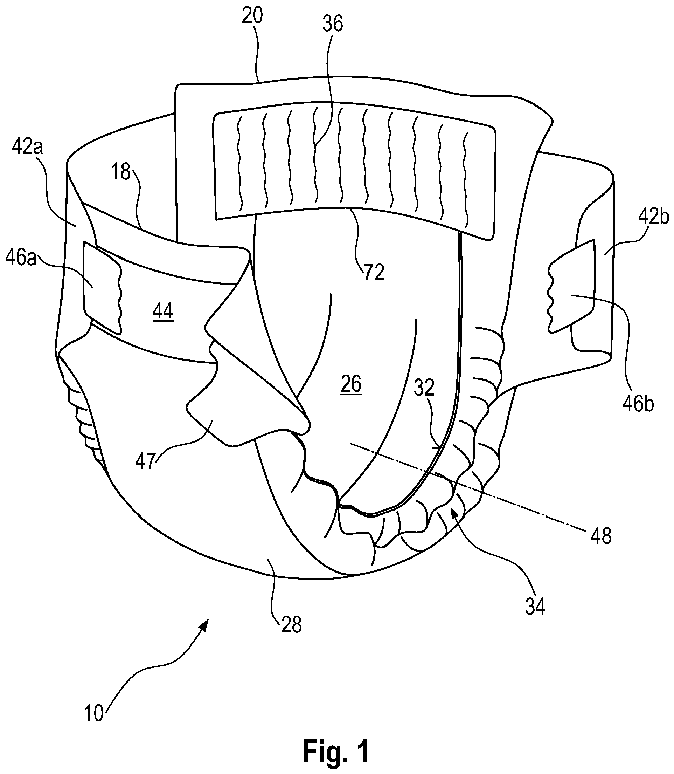

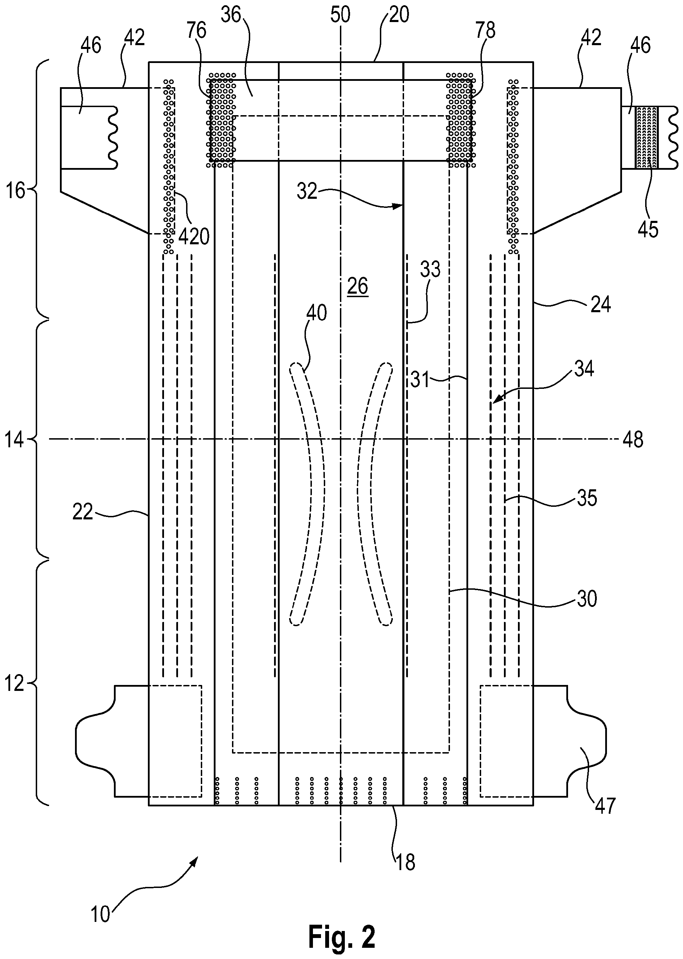

An example absorbent article 10 according to the present disclosure, shown in the form of a taped diaper, is represented in Fig. 1. Fig. 1 is a front perspective view of the absorbent article 10, with one of the back ears 42a having its fastening tab 46a fastened to the landing zone 44 and the other back ear 42b having its fastening tag 46b still unfastened. Fig. 2 is a plan view of the example absorbent article 10, wearer-facing surface facing the viewer, in a flat, laid-out state (i.e., the elastic contraction have been flattened out). The absorbent article 10 of Figs. 1-2 is shown for illustration purposes only as the present disclosure may be used for making a wide variety of diapers, including adult incontinence products, resealable pants, or other absorbent articles.

The absorbent article 10 comprises a liquid permeable topsheet 26, a liquid impermeable backsheet 28, and an absorbent core 30 positioned at least partially intermediate the topsheet 26 and the backsheet 28. The topsheet 26 forms the majority of the wearer-facing side of the article, and the backsheet 28 the majority of the garment-facing side of the article. These layers are discussed further below and together form at least partially the chassis of the article.

The absorbent article 10 comprises a front waist region 12, a crotch region 14, and a back waist region 16. The front waist region 12, the crotch region 14, and the back waist region 16 are each 1/3 of the length of the absorbent article 10 as measured along the longitudinal centerline 50. The crotch region 14 extends intermediate the front waist region 12 and the back waist region 16. The absorbent article 10 comprises a front edge 18, a back edge 20 opposite to the front edge 18, and longitudinally-extending, transversely opposed side edges 22 and 24 extending between the front edge 18 and the back edge 20. The outer perimeter dimensions of the article is substantially generally defined by the chassis.

Typically, diapers also comprise a pair of inner leg cuffs 32 that have an elasticized edge 33 that stand up and away from the chassis, and a pair of outer leg cuffs 34 with elastics 35 that are integrated in the chassis. The absorbent article 10 comprises a pair of back ears 42 in the back waist region 16 that extend transversally from the back waist region 16 of the absorbent article 10. The back ears 42 may comprise fastening tabs 46. The back ears 42 may be fastened (using the fastening tabs 46) to the landing zone area or landing zone material 44 disposed on a garment-facing portion of the front waist region 12 of the absorbent article 10. The absorbent article 10 may also have a front ear 47 on each side of the front waist region 12. The absorbent article 10 has a central longitudinal axis 50 that notionally divides the diaper symmetrically in a left half and right half. A transverse axis 48 that extends perpendicular to the central longitudinal axis 50 may also be notionally defined. Other components that are typically found in diapers are not shown from simplicity, but should be considered present for typical applications. In particular, absorbent articles typically comprise one or more acquisition layers positioned intermediate the topsheet 26 and the absorbent core 30 and are not further represented.

In other instances, the absorbent article may be in the form of a pant having refastenable side seams. Suitable refastenable seams are disclosed in US2014/0005020 and US9,421,137.

### Topsheet

The topsheet 26 is the part of wearer-facing side of the absorbent article 10 that is in contact with the wearer's skin. The topsheet 26 may be compliant, soft-feeling, and nonirritating to the wearer's skin. Further, at least a portion of, or all of, the topsheet may be liquid permeable, permitting liquid bodily exudates to readily penetrate through its thickness. A suitable topsheet may be manufactured from a wide range of materials, such as a nonwoven of an apertured plastic film, but other material are possible such as porous foams, reticulated foams, woven materials, woven materials. The nonwovens may comprise natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The topsheet 26 may be joined to portions of the backsheet 28, the absorbent core 30, the inner and outer leg cuffs 32, 34, and/or any other layers or components of the article as is known to those of ordinary skill in the art.

### Backsheet 28

The backsheet 28 is generally that portion of the absorbent article 10 positioned proximate to the garment-facing surface of the absorbent core 30. The backsheet 28 may be joined to portions of the topsheet 26, the outer cover material, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

An outer cover material (sometimes referred to as a backsheet nonwoven) may be joined to and cover the backsheet 28 on its garment-facing side. The outer cover material forms at least a portion of the garment-facing surface of the absorbent article 10 and effectively "covers" the backsheet 28 so that film is not present on the garment-facing surface. The outer cover material may comprise a bond pattern, apertures, and/or three-dimensional features. The outer cover material may be a hydroentangled nonwoven material.

### Absorbent Core 30

The term "absorbent core" refers to a component of the absorbent article 10 which can absorb and contain liquid such as urine received by the absorbent article. The absorbent core thus typically has a high absorbent capacity. The absorbent core comprises an absorbent material, which is typically enclosed within a core wrap. The absorbent core is shown is disposed between the topsheet and the backsheet and an exemplary outline is shown in dotted lines in Fig. 2. The chassis of the article is typically comprised by these layers which extend in the same longitudinal and transversal direction.

The core wrap may be a single material that is folded around the absorbent material, or it may comprise a separate top layer and bottom layer that may be bonded or otherwise joined together. The absorbent material typically comprises superabsorbent particles, optionally mixed with cellulose fibers. As used herein, "absorbent core" does not include any acquisition-distribution layer or system, topsheet, or backsheet of the absorbent article.

As illustrated, the outline of the absorbent core 30 may typically be generally rectangular, as defined by the longitudinal edges and transversal front edge and back edge of the core wrap. The absorbent material layer may also have a generally rectangular perimeter, but a non-rectangular perimeter cores are also commonly used, in particular the absorbent material may have a narrower width towards the central region of the core ("sand-hour" or "dog-bone" shape). In this way, the absorbent material area may have a relatively narrow width in an area of the core intended to be placed in the crotch region of the absorbent article. This may provide for example better wearing comfort. Other shapes can also be used such as a "T" or "Y" for the layer of absorbent material.

The absorbent material may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from 20 g/g to g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US 2008/0312617 and US2010/0051166A1 (both to Hundorf et al.) may be used. The present disclosure however is not limited to a particular type of absorbent cores. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in airfelt-free cores, as described in the above Hundorf references. These glues are not represented in the Figures for simplicity. Other core constructions comprising a high loft nonwoven substrate such as a carded nonwoven layer, having a porous structure into which SAP particles have been deposited, may also be used in present disclosure.

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area. As for example taught in US2008/312,622A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channel-forming areas 40, which are areas of the absorbent layer substantially free of absorbent material within the absorbent material layer. The top side of the core wrap may be advantageously bonded to the bottom side of the core by adhesive, mechanical or ultra-sonic bonding through these material-free areas. Example disclosures of such channels in an airfelt-free core can be found in WO2012/170778 (Rosati et al.) and US2012/0312491 (Jackels). Channels may of course also be formed in absorbent cores comprising a mix of cellulose fibers and SAP particles. These channels may embody any suitable shapes and any suitable number of channels may be provided. In other instances, the absorbent core may be embossed to create the impression of channels.

### Inner Leg Cuffs 32/ Outer Leg Cuffs 34

Referring to Figs. 1 and 2, for example, the absorbent article 10 may comprise one or more pairs of inner leg cuffs 32 and one or more pairs of outer leg cuffs 34. The inner leg cuffs 32 may be positioned laterally inboard of outer leg cuffs 34. Each of the inner leg cuff 32 may be formed by a piece of material which is partially bonded to a layer of the chassis of the absorbent article 10, and having an elasticized edge 33 which can stand upwards from the wearer-facing surface of the absorbent article 10. As is known in the art, inner leg cuffs (also designed as barrier leg cuffs) provide improved containment of body exudates approximately at the junction of the torso and legs of the wearer. The inner leg cuffs 32 are delimited by a proximal edge 31 joined directly or indirectly to the topsheet and/or the backsheet and a free terminal edge 33, which is intended to contact and form a seal with the wearer's skin. The inner leg cuffs 32 are at least present in the crotch region 14 and may extend at least partially to the front waist region 12 and back waist region 16 of the absorbent article 10 on opposite sides of the central longitudinal axis 50. The inner leg cuffs 32 may each comprise one or more elastics (e.g., elastic strands or strips) near or at the free terminal edge. These elastic strands provide the tension for the inner leg cuffs 32 to be raised vertically relative the wearer-facing side of the article, thus forming a seal around the thighs of the wearer. The outer leg cuffs 34 may also be present at least in the crotch portion 14 of the article, and may extend further to at least the front waist region 12 and the back waist region 16 of the article. The outer leg cuffs 34 comprise longitudinally extending elastic threads 35, which help the outer leg cuffs 34 to form a seal around the legs of the wearer at least in the article's crotch portion.

### Acquisition Materials

One or more acquisition materials (not represented for readability) are typically present at least partially intermediate the topsheet 26 and the absorbent core 30. The acquisition materials may be hydrophilic materials that provide significant wicking of the urine or other body exudates. These acquisition help dewatering the topsheet 26 to move the urine closer and into the absorbent core 30. The acquisition materials may comprise one or more nonwoven materials, foams, formed films, apertured formed films, cellulosic materials, cross-linked cellulosic materials, air laid cellulosic nonwoven materials, spunlace materials, or combinations thereof, for example. Typically, an acquisition material may have a width and length that are smaller than the width and length of the topsheet 26 and/or the absorbent core 30. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material oriented towards the topsheet, and as second acquisition material may comprise a cross-linked cellulosic material deposited on the first acquisition material and oriented towards the absorbent core.

### Landing Zone 44

Referring to Figs. 1 and 2, the absorbent article 10 may have a landing zone area 44 that is formed on a portion of the garment-facing surface of the outer cover material. The landing zone area 44 is typically the front waist region 12 of the absorbent article 10 as it fastens back to front. In some instances, the landing zone 44 may be or may comprise one or more discrete nonwoven materials that are attached to a portion of the backsheet 26 in the front waist region 12. In essence, the landing zone 44 is configured to receive the fasteners 45 disposed on the fastening tabs 46 that extend form the back ears 42. Common configurations comprise, for example, a plurality of loops on the landing zone 44 configured to be engaged with, a plurality of hooks on the fasteners 45, or vice versa.

### Wetness Indicator/Graphics

The absorbent articles of the present disclosure may comprise graphics and/or wetness indicators that are visible from the garment-facing surface (not represented for simplicity). The graphics may be printed on the landing zone , the backsheet, and/or at other locations. The wetness indicators are typically applied to the absorbent core facing side of the backsheet, so that they can be contacted by bodily exudates within the absorbent core. In some instances, the wetness indicators may form portions of the graphics. For example, a wetness indicator may appear or disappear and create/remove a character within some graphics. In other instances, the wetness indicators may coordinate (e.g., same design, same pattern, same color) or not coordinate with the graphics.

### Back ears 42 and front ears 47

The absorbent article 10 has a pair of back ears 42 disposed in the back portion on the article, and typically a pair of front ears 47 in the front portion in a taped diaper context. Only one pair of front and back ears may be required in most taped diapers. Each of the back ears 42 may comprise at its distal edge a discrete fastening tab 46 with fasteners 45 configured to engage the material in the landing zone 44. The back ears 42, or portions thereof, may be elastic or may have elastic panels. In an example, an elastic film or elastic strands may be positioned intermediate a first nonwoven material and a second nonwoven material. The elastic film may or may not be apertured. The back ears may be shaped. The back ears 42 are typically discrete components attached to a chassis of the absorbent article on a wearer-facing surface, on the garment-facing surface, or intermediate the two surfaces by attachment 420. The front ears are typically made of a nonwoven material having a basis weight of from 10 gsm to 30 gsm.

### Waist gasketing element 36

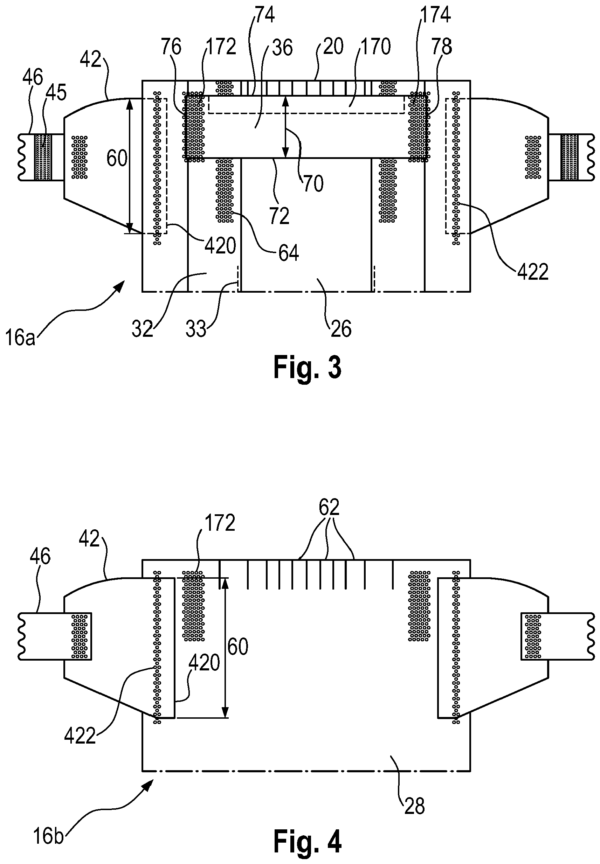

The absorbent article 10 comprise a waist gasketing element 36 disposed in the back portion 16 of the articles between the back ears 42. The waist gasketing element 36 provides improved fit and containment and typically may elastically expand and contract to dynamically fit a wearer's waist. The waist gasketing element 36 may be substantially rectangular with its long sides 72,74 oriented in the transversal direction, but the waist gasketing element is not necessarily a perfect rectangle. As showed in Fig. 3, the waist gasketing element 36 comprises a laterally-extending proximal end edge 72 and a longitudinally-opposing and laterally-extending distal end edge 74 (proximal and distal herein are relative to the transverse centerline 48 of the article). The waist gasketing element 36 may also have a first side edge 76 and a second side edge 78. Both side edges 76 and 78 extend longitudinally between the proximal end edge 72 and the distal end edge 74. The waist gasketing element 36 may be constructed in a number of different configurations including those described in US4,515,595 and US5,151,092.

It is to be appreciated that the waist gasketing element 36 may be located in various positions relative to various diaper components. For example, while the distal end edge 74 of the waist gasketing element 36 may be contiguous with the back waist edge 20 of the article, the waist gasketing element 36 may also be positioned longitudinally inwardly from the waist edges 20 of the absorbent article, as illustrated. For example, the longitudinal distance between the distal edge 74 of the waist gasketing element and the back edge 20 of the diaper 10 may be at least 5 mm. Such a distance may help avoiding that the waist gasketing element 36 extend over the back waist edge 20 of the article in case of misplacement in machine-direction during production.

The waist gasketing element 36 illustrated in Figs. 3-4 may be made as described in patent applications WO2020/242714, WO2021/092606, WO2020/242715 and WO2021/092607. While not limiting of the articles of the invention, the waist gasketing element 36 may be attached to the part of the chassis formed by the topsheet 26 by at least one adhesive disposed in an adhesive zone 170 and by one or more mechanical bonds 172, 174 disposed in corresponding non-adhesive zones. The adhesive zone 170 may be disposed adjacent the distal end edge 74 of the waist gasketing element 36, and the mechanical bonds 172,174 may be disposed on the first side edge 76 and a second side edge 78 respectively, as illustrated in Figure 3. Such embodiments are further disclosed in details in the four WO publications listed above, incorporated herein by reference.

The waist gasketing element 36 may comprise or consist of a laminate of an elastic film bonded between a first nonwoven and a second nonwoven. The first nonwoven, the elastic film and the second nonwoven may be bonded through any suitable techniques, such as ultrasonic bonding, adhesive bonding, and so forth. One or both of the nonwovens may be, for example, an elastomeric nonwoven or a non-elastic nonwoven. It is also appreciated that the first nonwoven and the second nonwoven may be the same size or may have different sizes and may define the same or different widths and/or lengths. Further, while the waist gasketing element may have two layers of nonwovens, it is to be appreciated that this disclosure is not so limited. For example, the continuous elastic substrate may be configured as a bi-laminate with an elastic film bonded with a single nonwoven substrate, such as bi-laminate formed with the elastic film and nonwoven. Further, instead of an elastic film, the waist gasketing element can comprise elastic strands, or other suitable materials. The laminate of film and one or more nonwovens can be an extrusion bonded laminate. It is also to be appreciated that the waist gasketing element may be assembled in various ways, such as for example, as disclosed in US6,572,595; US6,830,780; US7,087,287; and US 7,783,244; and US2018/0042778 A1; US2018/0042787 A1; US2018/0042779 A1; and US2018/0042778 A1, which are all incorporated by reference herein.

Mechanical bonds 76, 78 are shown in Figures 3-4 to depict the mechanical connection of the waist gasketing element 36 to the chassis (leg cuffs, topsheet and backsheet). In some configurations, however, the mechanical connection is between the waist gasketing element 36 and leg cuff 32 such that the topsheet is not incorporated into the mechanical bonds 76, 78. Furthermore, in some configurations, the waist gasketing element may be mechanically connected to the topsheet 26 but not the backsheet 28. Referring to Figures 3-4, mechanical bonds 76, 78 in the illustrated example are shown extending from the waist gasketing element 36 to the backsheet 28 and intermediate substrates therebetween, such as leg cuffs 32 and topsheet 26. A first grouping of mechanical bonds 76 may be positioned within a first adhesive-free zone and second grouping of mechanical bonds 78 may be positioned within a second adhesive-free zone. The first adhesive-free zone and the second adhesive-free zone may each have a width W1 in the transversal direction. In accordance with various configurations, the width W1 may be in the range of about 5 mm to about 30 mm, or may be in the range of about 10 mm to about 20 mm, or may be about 15 mm. Each of the first adhesive-free zone and the second adhesive-free zone may extend in the longitudinal direction at least from a proximal end edge 72 to a distal end edge 74 of the waist gasketing element 36. While mechanical bonds 76, 78 may extend through multiple absorbent article's components to bond the waist gasketing element 36 (as shown in Figs. 3-4), the mechanical bonds may extend through just the waist gasketing element 36 and the topsheet 26, or just through waist gasketing element 36 and the leg cuffs 32, or just through the waist gasketing element 36, the topsheet 26, and leg cuffs 32.

A central portion of the example waist gasketing element 36 is attached to the chassis 26 with an adhesive 170. The waist gasketing element 36 can define at least one adhesive zone 170 of adhesive on the surface of the waist gasketing element 36 that adheres to the chassis.

The waist gasketing element 36 is elasticized so that its forms wrinkles when no counter-acting tension is applied, as illustrated in Fig. 1. When the absorbent article is stretched flat as in Figs. 2-4, these wrinkles can be flattened out. The width of the waist gasketing element 36 under the tension required to stretch the article can be measured. Typically the width Wg of the waist gasketing element will range from 30 mm to 100 mm in these conditions. In an array according to the invention (as described below), the width Wg may be higher for the second article than for the first article. The width for a third article may be higher than for the second article.

When the fastening members 42 are pulled around the waist of the wearer and connected with a landing zone 44 on the front waist region to form a closed waist circumference, a direct line of tension is formed between the fastening members 42. Offsetting the axis of the waist gasketing element 36 from the axis of the tab 46 by a distance can beneficially keep the waist gasketing element 36 from being aligned with this direct line of tension. Thus, configuring the absorbent article 10 such that the waist gasketing element axis 36 is spaced away from the tab axis may allow for the waist gasketing element 36 to be exposed to less force levels than the levels of force to which the fastening members 42 are exposed. As a result, the stretchable waist gasketing element axis 36 can retain energy that can be used to close gaps when the wearer bends yet not impact the overall span of the users the article can fit.

Figs. 3-4 also show chassis end bonds 62 disposed at the back edge 20 of the diaper. The end bonds are mechanical or adhesive bonds that seal the absorbent article, in particular the topsheet and backsheet are sufficiently strongly bonded together by end bonds 62 to alleviate the risk that the layers of the chassis accidentally delaminate. Also shown in the Figures are tack-down bonds 64 that are typically heat or mechanical bonds that attach the inner leg cuff material 32 to the chassis, in particular the topsheet 26 in the back waist region of the article.

### Recycle Friendly and Bio-Based Absorbent Articles

Components of the absorbent articles described herein may at least partially be comprised of bio-based content as described in US2007/0219521A1. For example, the superabsorbent polymer component may be bio-based via their derivation from bio-based acrylic acid. Bio-based acrylic acid and methods of production are further described in US2007/0219521 and US 8,703,450; US9,630,901 and US9,822,197. Other components, for example nonwoven and film components, may comprise bio-based polyolefin materials. Bio-based polyolefins are further discussed in US2011/0139657, US2011/0139658, US2011/0152812, and US2016/0206772, and US9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about % to about 100%, from about 50% to about 100%, from about 75% to about 100%, or from about 90% to about 100%, for example, using ASTM D6866-10, method B.

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in US2019/0192723.

### Packages

The absorbent articles of the present disclosure may be placed into packages to be transported from the manufacturing site to the point of sales and/or the consumers. The package may comprise a polymeric film, carton, paper and/or any other common packaging materials. Graphics and/or indicia relating to properties of the absorbent articles may be formed on, printed on, positioned on, and/or placed on outer portions of the packages, in particular the size of the article and the corresponding weight range. Each package typically comprises a plurality of absorbent articles. The absorbent articles may be packed under compression so as to reduce the size of the packages, while still providing an adequate quantity of absorbent articles per package. By packaging the absorbent articles under compression, caregivers can easily handle and store the packages, while also providing distribution savings to manufacturers owing to the size of the packages.

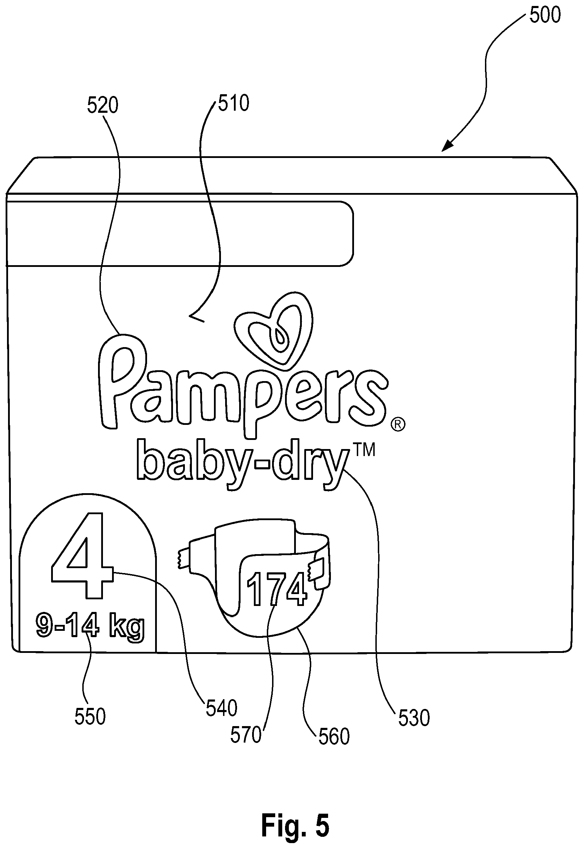

Fig. 5 illustrates a package 500 comprising absorbent articles. The package comprises at least one side 510 displaying information about the articles comprised in the package, such as the brand 520 of the article, e.g. Pampers^{®}, optionally a sub-brand 530 e.g. "baby-dry". The size 540 of the article, e.g. size 4, and the associated weight range 550 (e.g. 9-14 kg) are also typically indicated on at least the information side 510. An illustration 560 of the article may be typically included and an article count 570. As is known in the art, taped diapers for babies and infants have increasing weight ranges, which however can substantially overlap from one size to the size directly above, especially the upper limit of the range increases from one size to the next direct size.

### Array

"Array" means a pre-determined system of different absorbent articles having different constructions (e.g. different dimensions and/or absorbent capacity) and designed to be used in a certain order according to the different needs of the potential users. A typical array of absorbent articles according to the invention may in particular comprise a series of diapers adapted to be used progressively as the baby or infant develops. A plurality of absorbent articles of a given specification may be packaged in a package. The array of articles may thus be packaged in a corresponding array of packages. The packages have the same brand and/or sub-brand and/or the same trademark registration and may be available at a common point of sale (e.g. oriented in proximity to each other in a given area of a retail store). An array of articles of the invention are thus typically packaged in a series of packages having like packaging elements (e.g., packaging material type, film, paper, dominant color, design theme, etc.) that convey to consumers that the different individual packages are part of a larger line-up. Arrays often have the same brand, for example "Huggies", and same sub-brand, for example "Pull-Ups", or as illustrated in Figure 5 "Pampers" as main brand, and "baby-dry" as sub-brand. A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

### Array of absorbent articles according to the invention

An array of absorbent articles according to the invention comprises at least a first article and a second article, the second article being adapted for wearers having a larger size than the first article. The array of diapers typically comprise more than two articles, for example from 4 to 16 different articles, in particular from 6 to 14 different articles (including intermediate "P" or "plus" sizes if present, e.g. 4+). Each individual article is in the array is typically characterized by a size, which indicate the rank or order of that particular article within the array (typically a numbering is used, with a smaller number corresponding to a lower size) and an associated recommended weight range (typically the body weight in kg of the baby or infant recommended for that particular size). The manufacturer will adapt the dimensions and absorbent material quantity usage for each size according to the recommended user indicated.

As shown in Fig. 3, the back ears 42 each have an innermost edge 420, which is the edge closest to the longitudinal centerline 50, having a length 60 as measured in the longitudinal direction. The waist gasketing element 36 has a length 70 measured in the longitudinal direction (if the waist gasketing element is not rectangular, then the length is measured along the longitudinal axis 50). According to the invention, it was found that improved waist containment could be obtained when the ratio obtained by dividing the length 60 of the innermost edge of the back ears by the length 70 of the waist gasketing element should be in the range of 1.0 to 3.0, in particular from 1.5 to 2.5, more particularly from 1.8 to 2.2. Accordingly, in a first aspect of the invention, at least two articles of different size within the array have dimensions within this range. Preferably, at least 3, 4, 5, 6, 7 or 8 of the articles within the array have such ratio within the range of 1.0 to 3.0, in particular from 1.5 to 2.5, more particularly from 1.8 to 2.2. At least 50% of the articles within the array having a waist gasketing element (or at least 70%, or 100% of these articles within the array having a waist gasketing element) may have a ratio obtained by dividing the length 60 of the innermost edge of the back ears by the length 70 of the waist cuff element for each article in the range of 1.0 to 3.0, in particular from 1.5 to 2.5, more particularly from 1.8 to 2.2.

In another aspect, while the inventors have found that the ratio obtained by dividing the length of the innermost edge of the back ears by the length of the waist cuff element cuff may advantageously be in the range of 1.0 to 3.0, in particular from 1.5 to 2.5, more particularly from 1.8 to 2.2, it was also found that for better leakage prevention the length 70 of the waist gasketing element 36 should also increase with the size of the articles in the array. Not each and every sizes in the array requires an increase in this length compared to the size immediately below, however within the array of articles, the length of the waist gasketing element of a second article is at least 2 mm longer than the length of the waist gasketing element of a first article. This length difference may be at least 4 mm. For arrays spanning at least three different articles, or even more (e.g. an array of diaper extending from newborns to toddlers), a third article may be provided in the array, wherein the length of the waist gasketing element of the third article is at least 2 mm longer than the length of the waist gasketing element of the second article. The difference in the length may also be at least 4 mm longer.

In this context, the term "first" article, "second" article, and "third" article describe articles of different and increasing sizes, however the first, second and third size are not necessarily the size number 1, or size number 2 or size number 3 of the given array. Rather there may be several intermediate sizes between the first size, second size and the second size and the third size. This and different aspects of the invention are illustrated in the two exemplary arrays below.

### Example Array 1 (all dimensions in mm):

| | Size 1 | Size 2 | Size 3/3P | Size 4/4P | Size 5/5P | Size 6 | Size 7 | Size 8 |
|---|---|---|---|---|---|---|---|---|
| Recommended Weight Range (kg) | 2-5 | 4-8 | 6-10(+1) | 9-14(+1) | 11-15(+1) | 13+ | 15+ | 17+ |
| Back Ear Innermost Edge Length (60) | 81 | 81 | 92 | 92 | 108 | 118 | 118 | 118 |
| Waist Gasketing Element Length (70) | 40 | 40 | 45 | 45 | 50 | 50 | 50 | 50 |
| Ratio (60/70) | 2.03 | 2.03 | 2.04 | 2.04 | 1.96 | 2.15 | 2.15 | 2.15 |

Example array 1 comprises 8 basic sizes and 3 additional plus sizes ("P"), thus forming an array comprising 11 articles of increasing recommended weight range. The P size have a higher limit for their weight range boundary (+1 = +1 kg) compared to the base size that they extend. Each of the articles has a ratio obtained by dividing the length of the innermost edge of the back ears by the length of the waist cuff element cuff in the range of 1.0 to 3.0, in particular from 1.5 to 2.5, more particularly from 1.8 to 2.2. The articles have three different Waist Gasketing Element Length (40 mm, 45 mm, 50 mm), which increase with the size of the articles. Thus in this, the size 1-3 articles are each a first article according to the invention, and any of the size 3-4 article are a second article according to the invention, while any of the size 5-8 articles are a third article of the invention, with the ratio 60/70 for these third articles being in the range claimed and the Back Ear Innermost Edge Length also being 5 mm longer than the Back Ear Innermost Edge Length of the second article. Alternatively, the articles of size 5 and above may have a longer Waist Gasketing Element Length of 55 mm instead of 50 mm for even more protection with acceptable additional material cost.

### Example Array 2 (all dimensions in mm):

| | Size 1 | Size 2 | Size 3/3P | Size 4/4P | Size 5/5P | Size 6 | Size 7 | Size 8 |
|---|---|---|---|---|---|---|---|---|
| Recommended Weight Range (kg) | 2-5 | 4-8 | 6-10(+1) | 9-14(+1) | 11-15(+1) | 13+ | 15+ | 17+ |
| Back Ear Innermost Edge Length (60) | 81 | 81 | 93 | 109 | 118 | 118 | 118 | 118 |
| Waist Gasketing Element Length (70) | 40 | 40 | 45 | 45 | 50 | 50 | 50 | 50 |
| Ratio (60/70) | 2.03 | 2.03 | 2.07 | 2.42 | 2.15 | 2.15 | 2.15 | 2.15 |

The example array 2 also comprises 8 basic sizes and 3 additional plus sizes ("P"), thus forming an array comprising 11 articles of increasing recommended weight range. Each of the articles has a ratio obtained by dividing the length of the innermost edge of the back ears by the length of the waist gasketing element cuff in the range of 1.0 to 3.0, in particular from 1.5 to 2.5, and 7 out of 8 articles have a ratio in the range of from 1.8 to 2.2. The articles have three different Waist Gasketing Element Length (40 mm, 45 mm, 50 mm). The sizes 1-2 articles are each a first article according to the invention (length 70 = 40 mm), while each of the size 3/P-4/P articles are a second article according to the invention (length 70 = 45 mm), and each of the articles of sizes 5 and above are a third article according to the invention (length 70 = 50 mm). Optionally, the articles of size 5 and above may have a longer Waist Gasketing Element Length of 55 mm instead of 50 mm for even more protection with acceptable additional material cost.

### Misc

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as " mm" is intended to mean "about mm."

## Claims

1. An array of personal hygiene articles (10) comprising at least a first article and a second article, the second article being adapted for wearers having a larger size than the first article,
each article comprising a longitudinal centerline (50), a transversal centerline (48), a chassis comprising a topsheet (26), a backsheet (28), an absorbent core (30), optionally an acquisition material, a pair of back ears (42) attached to and extending transversally outwardly from the chassis, and a waist gasketing element (36) disposed on the wearer-facing side of the article between the back ears (42);
wherein the back ears (42) each have an innermost edge (420) having a length (60); wherein the waist gasketing element (36) has a length (70) measured along the longitudinal centerline;
wherein the ratio of the length (60) of the innermost edge of the back ears to the length of the waist gasketing element (70) is in the range of from 1.0 to 3.0, for at least the first article and the second article; and wherein the length of the waist gasketing element of the second article is at least 2 mm longer than the length of the waist gasketing element of the first article, and preferably at least 4 mm longer.

2. An array of personal hygiene articles according to claim 1, wherein the ratio of the length (60) of the innermost edge of the back ears to the length of the waist gasketing element (70) is in the range of from 1.5 to 2.5, more preferably 1.8 to 2.2 for at least the first article and the second article.

3. An array of personal hygiene articles according to claim 1 or 2, the array comprising at least a third article, the third article being adapted for wearers having a larger size than the second article;
wherein the ratio of the length of the innermost edge of the back ears to the length of the waist gasketing element for the third article is in the range of from 1.0 to 3.0, preferably 1.5 to 2.5, more preferably 1.8 to 2.2; and
wherein the length of the waist gasketing element of the third article is at least 2 mm, preferably at least 4 mm, longer than the length of the waist gasketing element of the second article.

4. An array of personal hygiene articles according to any of the preceding claims, the array comprising from 3 to 12 articles of different sizes, and wherein at least 50% of the articles in the array have a waist gasketing element.

5. An array of personal hygiene articles according to any of the preceding claims, wherein the length of the waist gasketing element of the first article, second article and if present the third article, is between 30 mm and 60 mm for each of these articles, the length being measured in the longitudinal direction.

6. An array of personal hygiene articles according to the preceding claim, wherein the length of the waist gasketing element of the first article is in the range of from 30 mm to 50 mm; and the length of the waist gasketing element of the second article is in the range of from 35 mm to 55 mm; and the length of the waist gasketing element of the third article if present is in the range of from 40 mm to 60 mm.

7. An array of personal hygiene articles according to any of the preceding claims, wherein the width of the waist gasketing element for at least the first article, the second article and the third article if present is in the range of from 30 mm to 100 mm, the width being measured in the transversal direction with the waist gasketing element being gently stretched to remove any wrinkles.

8. An array of personal hygiene articles according to any of the preceding claims, wherein at least one, or more than one, or all of the articles in the array have at least one channel-forming area (40) in the absorbent core.

9. An array of personal hygiene articles according to any of the preceding claims, wherein at least one, or more than one, or all of the articles in the array has an absorbent core which is substantially free of cellulose fibers in the absorbent material.

10. An array of personal hygiene articles according to any of the preceding claims, wherein at least a portion of the waist gasketing element (36) is adhesively attached to the chassis in an adhesive zone (170), and a least a portion of the waist gasketing element comprises a mechanically attachment (172, 174) to the chassis in a first adhesive-free zone disposed at and adjacent to a first side edge (76) of the waist gasketing element (36) and in a second adhesive-free zone is disposed at and adjacent to a second side edge (78) of the waist gasketing element.

11. An array of personal hygiene articles according to the preceding claim, wherein the waist gasketing element comprises a third adhesive-free zone disposed at and adjacent to the proximal end edge (72) of the waist gasketing element at a central region of the waist gasketing element, such that the proximal end edge of the waist gasketing element at the central region is not attached to the chassis.

12. An array of personal hygiene articles according to any of claims 10-11, wherein the adhesive zone (170) is disposed at and adjacent to the distal end edge (74) of the waist gasketing element.

13. An array of personal hygiene articles according to any of claims 10-12, wherein the first and second mechanical attachments (172, 174) extend through the waist gasketing element, the topsheet, and the backsheet.

14. An array of personal hygiene articles according to any of the preceding claims, wherein the array comprises distinct packages (500) for each of the articles of a different size (540) in the array.

15. An array of personal hygiene articles according to any of the preceding claims, wherein the personal hygiene articles are taped diapers for baby and/or infants with different recommended weight ranges (550).
